# EUROPEAN PATENT APPLICATION

(11) **EP 1 860 105 A1**
(43) Date of publication of application: **28.11.2007**
(21) Application number: 07010347.8
(22) Date of filing: 24.05.2007
(51) Int. Cl.: C07D 401/04

(54) **Process for the preparation of desloratadine**

(30) Priority: 24.05.2006 IN DE12502006
(71) Applicant: Ranbaxy Laboratories Limited, Gurgaon, Haryana 122001 (IN)
(72) Inventor: Rafeeq, Mohammad, Uttar Pradesh 262121 (IN); Agrawal, Ashul, Gurgaon, Haryana 122001 (IN); Murugesan, Balaguru, Gurgaon, Haryana 122001 (IN); Sathyanarayana, Swargam, Andhra Pradesh 505002 (IN)
(74) Representative: Cronin, Brian Harold John

(57) **Abstract**

The present invention relates to novel processes for the preparation of Form 2 of desloratadine.

## Description

### Field of the Invention

The present invention relates to processes for the preparation of Form 2 of desloratadine.

### Background of the Invention

Desloratadine, 8-Chloro-6, 11-dihydro-11- (4-piperidinylidene)-5H-benzo[5,6] cyclohepta [1,2-b] pyridine, is represented by Formula I

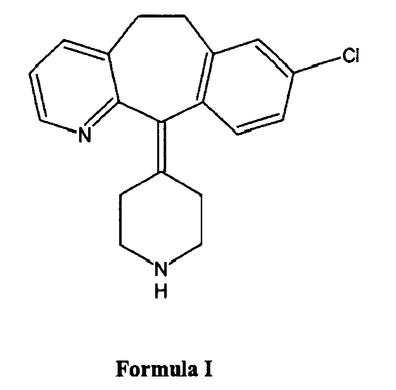

Desloratadine is a metabolite of loratadine, having non-sedative antihistaminic activity and is known from U.S. Patent No. 4,659,716. Several methods for the preparation of desloratadine are known in the literature, such as those described in, for example, U.S. Patent Nos. 5,151,423; 4,873,335; and 5,595,997; U.S. Publication Nos. 2004/0242619; and 2005/0131046, and PCT Publication Nos. WO 03/086275, WO 2004/029039, WO 2004/029039, which are herein incorporated by reference.

Three different polymorphs of desloratadine, Form 1, Form 2 and an amorphous form, are known in the literature.

WO 2005/084674 describes an amorphous form of desloratadine and a process for its preparation.

U.S. Patent No. 6,506,767 describes a crystalline polymorph Form 1 of desloratadine essentially free of polymorph Form 2 and polymorph Form 2 of desloratadine substantially free of polymorph Form 1. This patent describes the use of specific solvents such as alcohols, chlorinated hydrocarbons, ethers and ketones to produce 100% pure crystalline Form 1 of desloratadine, Form 1 substantially free of Form 2 and Form 1 having significant amount of Form 2. This patent also describes the preparation of crystalline Form 2 substantially free of Form 1 of desloratadine, by precipitation from a reaction mixture containing a solution of desloratadine in di-n-butyl ether, and of pure Form 2 by refluxing desloratadine in ethyl acetate followed by hot filtration and rapid cooling of the reaction mixture.

WO 2004/080461 describes the preparation of mixtures of Form 1 and Form 2 of desloratadine. It describes that Form 2 having substantial amount of Form 1 may be obtained by addition of a small amount of 2-propanol to a solution of desloratadine in toluene whereas substantially Form 2 is obtained when only toluene is used without adding 2-propanol .

U.S. Publication No. 2004/0242619 describes a process for the preparation of crystalline Form 2 of desloratadine comprising the steps of melting desloratadine, cooling the molten desloratadine to obtain a solid and grinding the solid. It also describes the preparation of Form 2 of desloratadine by precipitation from a solution in dimethylcart onate or by crystallization from toluene.

The present invention provides processes for the preparation of Form 2 of desloratadine by spray drying or agitated thin film drying.

### Summary of the Invention

In on 3 aspect, a process for the preparation of Form 2 of desloratadine is provided comprising:
a) providing a solution of desloratadine in a suitable solvent, and
b) recovering Form 2 of desloratadine by spray drying.

In another aspect, a process for the preparation of Form 2 of desloratadine is provided comprising:
a) providing a solution of desloratadine in a suitable solvent, and
b) recovering Form 2 of desloratadine by agitated thin film drying technique.

In a further aspect, Form 2 of desloratadine prepared by a process according to a first aspect, having an X-ray diffraction pattern, for example, as depicted in Figure I is provided.

In ye: another aspect, Form 2 of desloratadine prepared by a process according to a first aspect, having an IR spectrum, for example, as depicted in Figure II is provided.

In still another aspect, Form 2 of desloratadine prepared by a process according to a second aspect, having an X-ray diffraction pattern, for example, as depicted in Figure III is provided.

In ye: a further aspect, Form 2 of desloratadine prepared by a process according to a second aspect, having an IR spectrum, for example, as depicted in Figure IV in described.

### Brief Description of the Figures

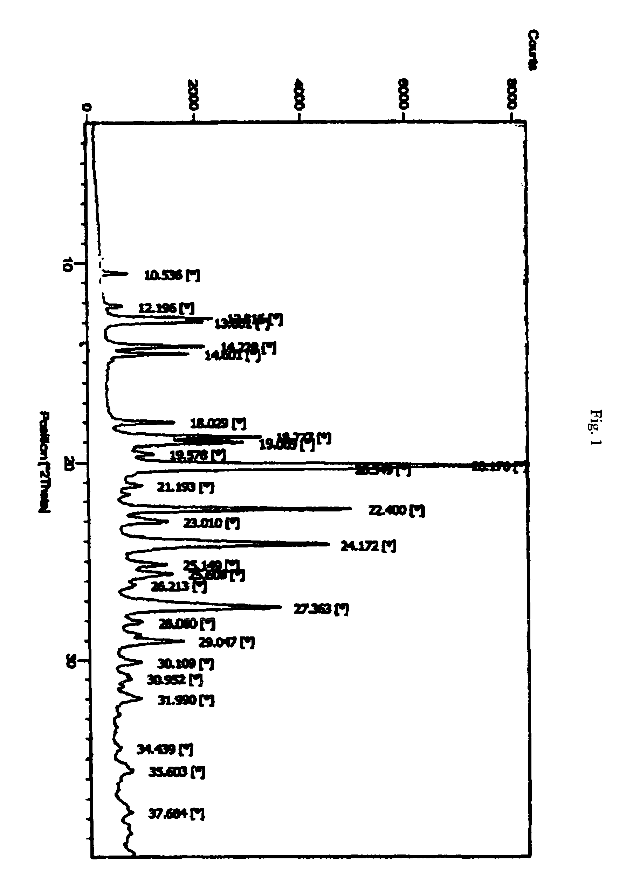

Fig. 1 is an X-ray diffraction pattern of Form 2 of desloratadine obtained by spray drying.
Fig. II is an IR spectrum of Form 2 of desloratadine obtained by spray drying.
Fig. III is an X-ray diffraction pattern of Form 2 of desloratadine obtained by agitated thin film drying.
Fig. IV is an IR spectrum of Form 2 of desloratadine obtained by agitated thin film drying.

### Detailed Description of the Invention

Desloratadine used for the preparation of Form 2 of desloratadine can be obtained by any of the methods known in the art, for example, including those described in U.S. Patent Nos. 5,151,423; 4,873,335; or 5,595,997, U.S. Publication Nos. 2004/0242619, or 2005/0131046, or PCT Publication Nos. WO 03/086275, WO 2004/029039, or WO 2004/029039. The starting desloratadine may be obtained as a solution directly from a reaction mixture in which desloratadine is formed and used as such without isolation.

Suitable solvents used to prepare the solution of desloratadine may include organic solvents or mixtures of organic solvents, with or without water.

The organic solvent(s) may be selected from, for example, alkyl acetates, dipolar aprotic solvents and/or mixtures thereof. Examples of alkyl acetate may include ethyl acetate, n-propyl acetate, n-butyl acetate, iso-propyl acetate, iso-butyl acetate and the like. Examples of dipolar aprotic solvents may include acetonitrile, dimethylformamide, dimethylsulphoxide and the like. In some embodiments, mixtures of ethyl acetate and water can be used.

The mixture of desloratadine in organic solvent and water may be filtered to remove any un-dissolved foreign particulate matter.

Deslc ratadine may be added to a mixture of organic solvent and water at a temperature of about 15-50°C. In some embodiments, desloratadine may be added at a temperature of, for example, about 30-35°C.

Wate may be separated from the mixture by azeotropic distillation or by layer separation to obtain a solution of desloratadine in organic solvent.

A spray dryer may be used for spray drying. The spray dryer operates on the principle of r ozzle spraying in a parallel flow, i.e., the sprayed product and the drying gas flow in the same direction. The drying gas can be air or inert gas such as nitrogen, argon or carbon dicxide. In some embodiments, the drying gas can be nitrogen.

The f ow rate may range from about 750-900 mL/ hour. In some embodiments, the flow rate can be about 825 mL/hour. The spray drying may be carried out at an inlet temperature of about 120-160°C and at an outlet temperature of about 90-120°C. In some embodiment, the inlet temperature can be about 130°C and the outlet temperature can be about 100°C.

Agitated thin film drying involves separating the volatile component using indirect heat transfer coupled with mechanical agitation of the flowing film under controlled condition. In some embodiments, a vertical agitated thin film dryer can be used.

In vertical agitated thin-film drying, the starting material is fed from the top into a cylindrical space between a centered rotary agitator and an outside heating jacket. The rotor agitates the downside flowing solution while the heating jacket heats it.

The heating jacket may be maintained at a temperature of, for example, about 75-110°C. In some embodiments, the heating jacket can be maintained at a temperature of about 90-92°C.

In the foregoing section embodiments are described by way of examples to illustrate the process of invention. However, this is not intended in any way to limit the scope of the present invention. Variants of the examples would be evident to persons ordinarily skilled in the art.

### Methods

### Powder XRD

X-Ray Difractometer, Rigaku Coorperation, RU-H3R
Goniometer CN2155A3
X-Ray tube with Cu target anode
Divergence slits 1 0, Receiving slit 0.15mm, Scatter slit 1 0
Power: 40 KV, 100 mA
Scanning speed: 2 deg/min step: 0.02 deg
Wave length: 1.5406 A

### Spray Dryer: PST 01

### Example 1: Preparation of Form 2 of Desloratadine by Spray drying

Desloratadine (100 g) was dissolved in a mixture of ethyl acetate (1000 mL) and deioniozed water (100 mL) at a temperature of 30-35°C. The solution was filtered through a hyflobed. The organic layer was separated and fed into a spray dryer in an atmosphere of nitrogen. The inlet temperature was kept at 130°C and the outlet temperature was kept at 100°C for 1 hour 20 minutes.
Flow Rate: 8 25 mL/hour, Yield: 0.5 w/w, HPLC purity: 99.53%.

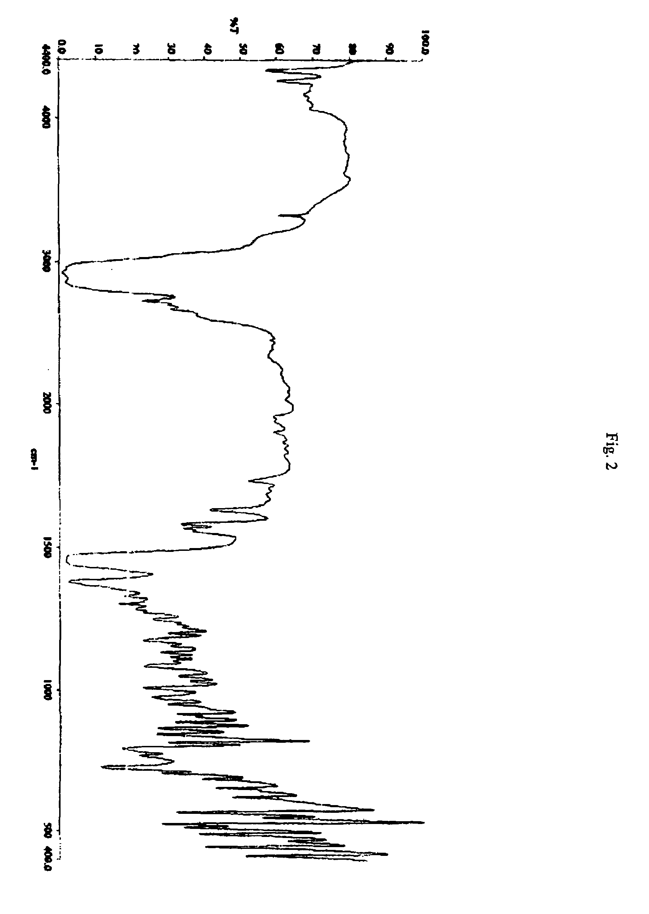

This material was used to generate an X-ray diffraction spectrum as shown in Fig. 1 and an IR spectrum as shown in Fig. 2.

### Example 2: Preparation of Form 2 of Desloratadine by Agitated Thin Film Drying

Desloratadine (10g) was dissolved in a mixture of ethyl acetate (50 mL) and deioniozed water (10 mL) at a temperature of 30-35°C. The organic layer was separated and fed into an agitated thin film dryer at a temperature of 90-92°C for 5-6 hours under reduced pressure.
Yield: 7.6 g, HPLC purity: 99.71%.

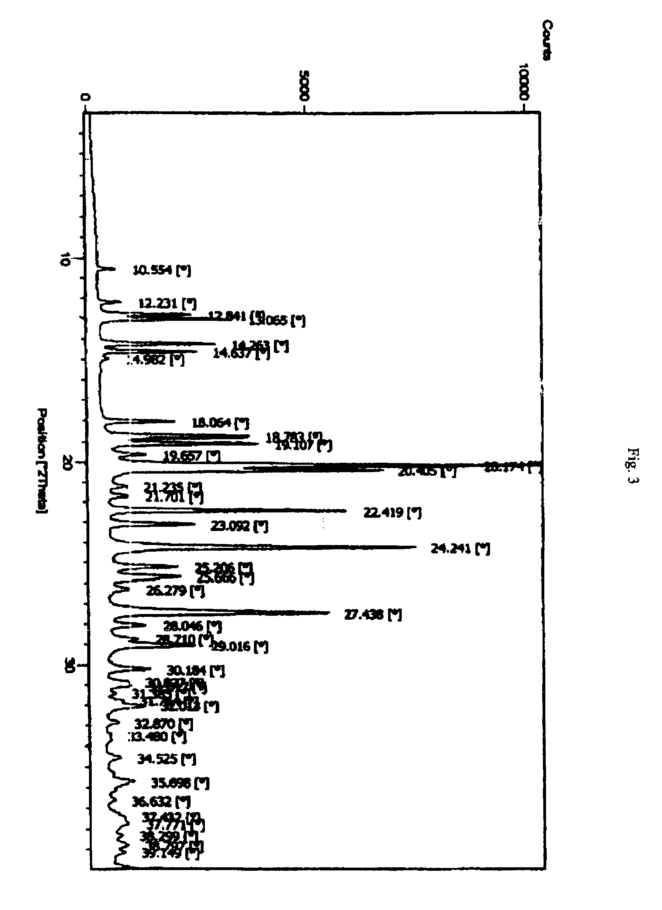

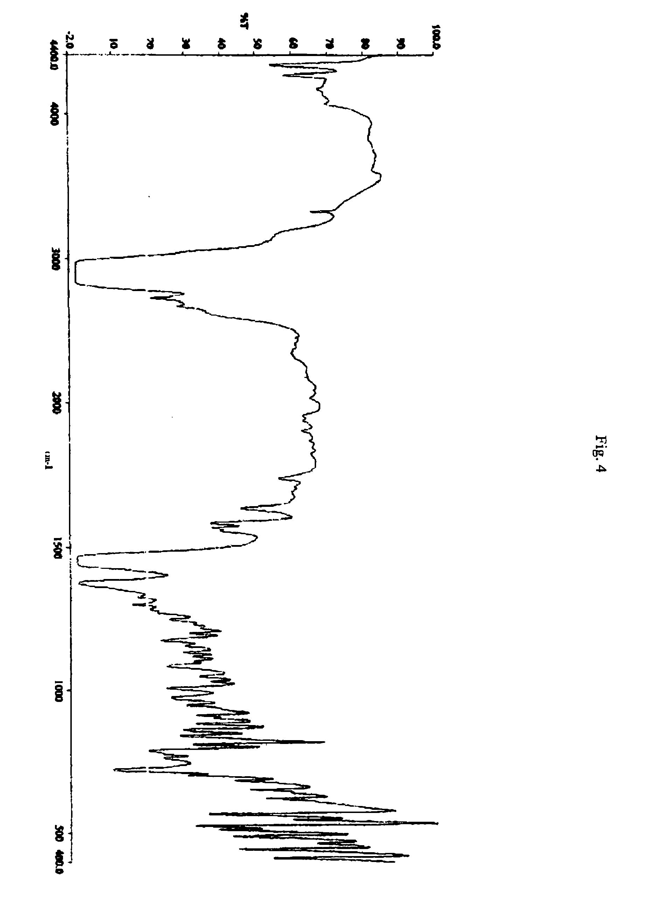

This material was used to generate an X-ray diffraction spectrum as shown in Fig. 3, and an IR spectrum as shown in Fig. 4.

## Claims

1. A process for the preparation of Form 2 of desloratadine comprising:
a) providing a solution of desloratadine in a suitable solvent, and
b) recovering Form 2 of desloratadine by spray drying.

2. A process for the preparation of Form 2 of desloratadine comprising:
a) providing a solution of desloratadine in a suitable solvent, and
b) recovering Form 2 of desloratadine by agitated thin film drying.

3. Form 2 of desloratadine obtained by the process of claim 1 having XRD pattern as depicted in Figure I.

4. Form 2 of desloratadine obtained by the process of claim 1 having IR as depicted in Figure II.

5. Form 2 of desloratadine obtained by the process of claim 2 having XRD pattern as depicted in Figure III.

6. Form 2 of desloratadine obtained by the process of claim 2 having IR as depicted in Figure IV.

7. The process according to claims 1 or 2, wherein desloratadine, obtained as a solution directly from a reaction mixture in which desloratadine is formed, is used as such without isolation.

8. The process according to claim 1, wherein the spray drying is carried out at an inlet temperature about 120-160°C.

9. The process according to claim 1, wherein Form 2 is recovered from the spray dryer at an outlet temperature of about 90-120°C.

10. The process according to claim 2, wherein a vertical agitated thin film dryer is used.
